(19) Europäisches Patentamt
European Patent Office
Office européen des brevets

(11) Publication number: 0 492 430 A1

# (12) EUROPEAN PATENT APPLICATION

(21) Application number: 91121765.1

(22) Date of filing: 19.12.91

(51) Int. Cl.[5]: C07H 19/02, A61K 31/70

(30) Priority: 27.12.90 JP 415126/90

(43) Date of publication of application: 01.07.92 Bulletin 92/27

(84) Designated Contracting States: DE FR GB IT

(71) Applicant: NIPPON KAYAKU KABUSHIKI KAISHA
11-2, Fujimi 1-chome Chiyoda-ku
Tokyo 102(JP)

(72) Inventor: Saito, Seiichi
4-7-2-407, Matsubacho
Kashiwa-shi, Chiba-ken(JP)
Inventor: Kitagawa, Masayuki
293, Inumakata, Ouaza
Urawa-shi, Saitama-ken(JP)
Inventor: Masuda, Akira
1039, Kamiochiai
Yono-shi, Saitama-ken(JP)
Inventor: Hasegawa, Shigeru
293, Inumakata, Ouaza
Urawa-shi, Saitama-ken(JP)
Inventor: Abe, Fuminori
3-29-5, Shimo, Kita-ku
Tokyo(JP)

(74) Representative: Türk, Gille, Hrabal
Brucknerstrasse 20
W-4000 Düsseldorf 13(DE)

## (54) Oxetanocin derivative having imidazole base group.

(57) The present invention relates to an oxetanocin derivative having an imidazole base group which is represented by the following general formula (A):

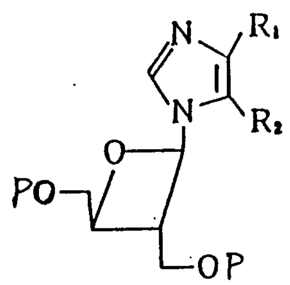

(A)

wherein $R_1$ represents a carbamoyl group or a cyano group;
$R_2$ represents an amino group, an acetylene group or a halogen atom; and
P represents a hydrogen atom or a protective group.

The compound of the present invention, which acts on an immune system, is useful as an active ingredient of an immunosuppressant. Further, useful intermediates of this compound are disclosed.

EP 0 492 430 A1

Background of the Invention:

It is expected that the compound of the present invention exerts physiological activities including an immunosuppressive effect or is useful as an intermediate for synthesizing such a physiologically active substance.

Statement of the Prior Art:

Conventional immunosuppressants include steroid compounds, those acting on the synthesis of nucleic acids, for example, azathiopurine, and antibiotics, for example, cyclosporin A. There has been urgently required, however, to develop a novel compound having an immunosuppressive effect, since the known ones are apt to exert side effects.

There have been disclosed oxetanocin derivatives having a purine base group in, for example, EP-A2-0291917 and EP-A2-0334250. It is expected that these compounds are applicable to drugs such as antiviral agents.

However no oxetanocin derivative having an imidazole base group has been known so far. Summary of the Invention:

The present invention provides an oxetanocin derivative having an imidazole base group which has an immunosuppressive activity and an oxetanocin derivative having an imidazole base group which is useful as an intermediate of the aforesaid compound. Accordingly, the present invention relates to an oxetanocin derivative having an imidazole base group which is represented by the following general formula (A):

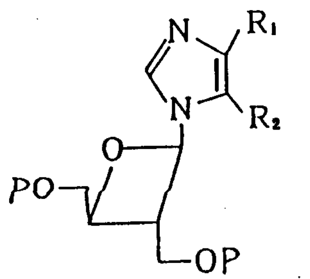

(A)

wherein $R_1$ represents a carbamoyl group or a cyano group;
$R_2$ represents an amino group, an acetylene group or a halogen atom; and
P represents a hydrogen atom or a protective group.

The compound of the present invention is useful as an immunosuppressant or an intermediate thereof.

Namely, the compound of the present invention wherein $R_1$ is a cyano group and $R_2$ is an acetylene group is useful as an immunosuppressant, while other compounds of the present invention are useful each as an intermediate thereof.

Detailed Description of the Invention:

In the present invention, P represents a hydrogen atom or a protective group of a hydroxyl group. Thus any protective group commonly used for protecting a hydroxyl group of a nucleic acid may be selected therefor. Examples thereof include acyl groups having 1 to 10 carbon atoms, such as acetyl, chloroacetyl, dichloroacetyl and benzoyl groups, substituted and unsubstituted benzyl groups and silyl groups having one, two or three hydrocarbon substituents each having 1 to 10 carbon atoms, such as trimethylsilyl, triethylsilyl and t-butyldimethylsilyl groups. It is particularly preferable in the present invention to use an acyl group therefor.

Examples of the halogen atom of $R_2$ include fluorine, chlorine, bromine and iodine atoms. It is particularly preferable in the present invention to use bromine or iodine atom therefor.

Typical examples of the compound of the present invention are as follows:

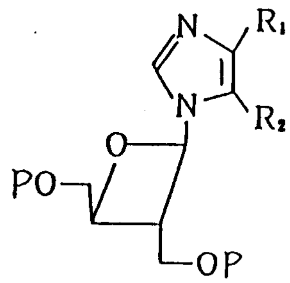

(A)

| Compound No. | $R_1$ | $R_2$ | P |
|---|---|---|---|
| (1) | $CONH_2$ | $NH_2$ | H |
| (2) | $CONH_2$ | $NH_2$ | $COCH_3$ |
| (3) | CN | $NH_2$ | $COCH_3$ |
| (4) | CN | $NH_2$ | H |
| (5) | CN | C≡CH | $COCH_3$ |
| (6) | CN | C≡CH | H |
| (7) | CN | I | $COCH_3$ |

Next, methods for producing the compounds of the present invention will be described.

The compound of the present invention may be obtained from oxetanocin A N-oxide (①) which has been disclosed in, for example, Japanese Patent Laid-Open No. 279693/1990.

Now the steps for synthesizing each compound of the present invention will be described.

1. Synthesis of compound No. 1 [formula (A): $R_1$ = $CONH_2$, $R_2$ = $NH_2$, P = H]:

It is obtained from a known compound oxetanocin A N-oxide (①) via the synthesis pathway given in Scheme 1. In the compound of the formula (②), X is a halogen atom, preferably a bromine atom in the present invention. $P_1$ is an alkyl group derived from a hydrocarbon having 1 to 10 carbon atoms, such as a methyl, ethyl, t-butyl, hexyl or benzyl group, preferably a t-butyl or benzyl group in the present invention.

Now the synthesis of the compound No. 1 of the present invention will be described in two steps.

1-1. Compound (①) (oxetanocin A N-oxide) → compound of the formula (③) ($P_1$ = lower alkyl group):

The compound (①) is suspended or dissolved in an organic solvent such as N,N-dimethylacetamide or N,N-dimethylformaide and an aralkyl halide such as benzyl bromide or an alkyl halide such as t-butyl bromide is added thereto. Then the mixture is allowed to react at a temperature of from 0 °C to the boiling point of the solvent, preferably at approximately 10 to 40 °C, for 4 to 48 hours, preferably for 8 to 24 hours. After adding water to the reaction mixture, the organic solvent is extracted with, for example, chloroform. The aqueous phase is concentrated to thereby remove the water. Thus a reaction product of the formula (② ) (X = halogen ion, $P_1$ = lower alkyl group) is obtained as a syrup. Then this syrupy product is reacted with an aqueous solution of an alkali hydroxide, for example, sodium hydroxide, or an aqueous solution of ammonia or triethylamine, preferably an aqueous solution of an alkali hydroxide, at 5 to 50 °C, preferably 10 to 40 °C, for 4 to 48 hours, preferably for 8 to 24 hours. The reaction product thus obtained by the above ring-opening reaction is isolated by, for example, column chromatography to thereby give a compound of the formula (③) wherein $P_1$ is a lower alkyl group.

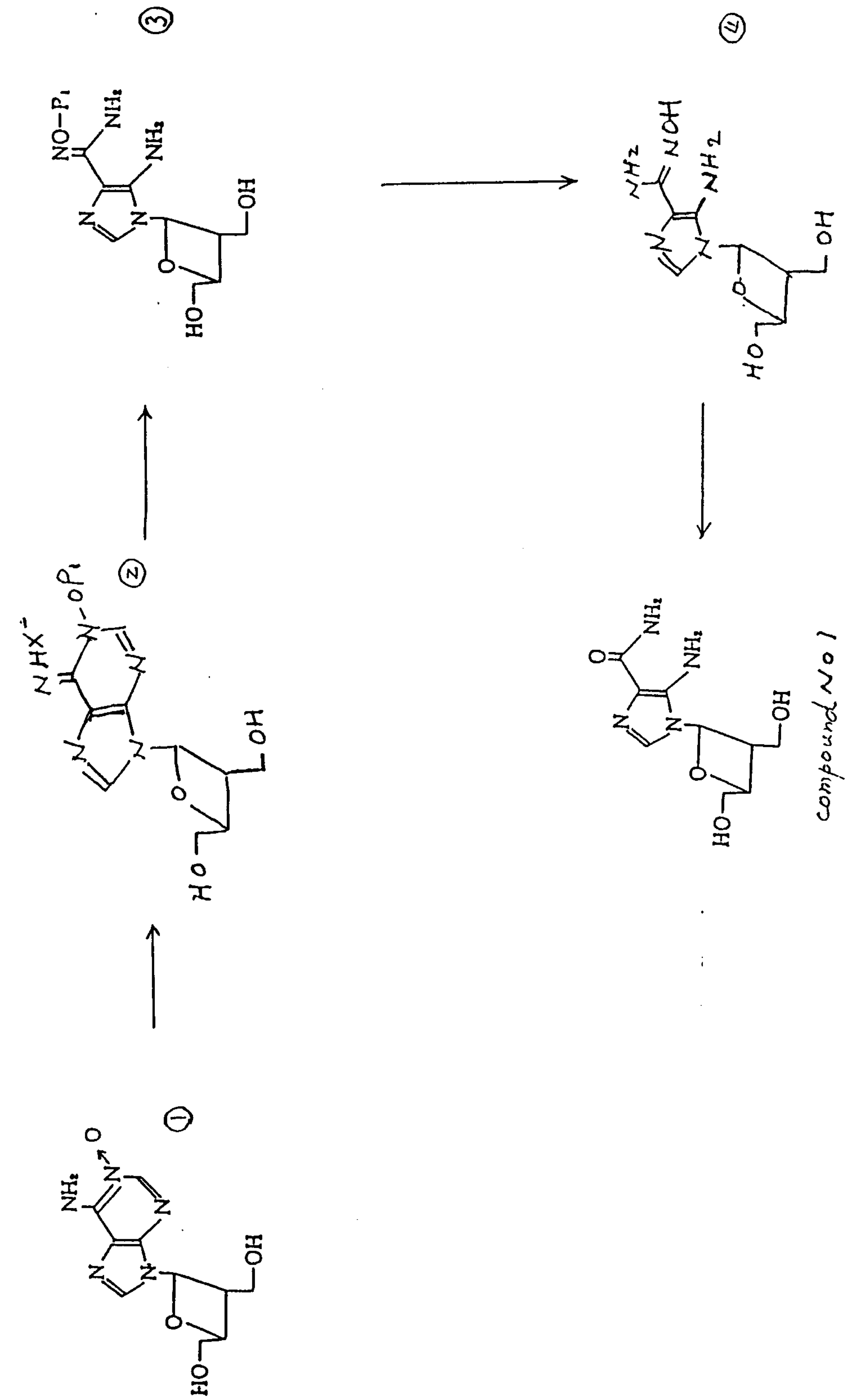

①

②

③

④

compound No 1

Scheme 1: Synthesis pathway of compound No. 1

1-2. Compound of formula (③) ($P_1$ = lower alkyl group) → compound No. 1:

The compound of the formula (③) wherein $P_1$ is a lower alkyl group is dissolved in a polar organic solvent such as a lower alcohol, for example, methanol or ethanol, and then subjected to hydrogenolysis in the presence of a noble metal catalyst, for example, Pd-C. This reaction may be carried out at 0 to 60 °C,

preferably approximately 10 to 40°C. Although this reaction may be effected under atmospheric pressure, it is preferable in general to conduct the reaction under elevated pressure, approximately 1 to 5 atm. After removing the catalyst and the solvent from the reaction mixture, concentrated aqueous ammonia is added to the residue [i.e., a compound of the formula (④)] and the reaction vessel is sealed. Next, the mixture is allowed to react by heating to approximately 40 to 150°C, preferably 60 to 120°C, for 1 to 5 days, preferably 2 to 4 days. Then the reaction product is isolated by, for example, chromatography to thereby give the compound No. 1.

The compound No. 1 is useful as an intermediate for synthesizing the compound No. 6.

2. Synthesis of compound No. 2 [formula (A): $R_1$ = $CONH_2$, $R_2$ = $NH_2$, P = $COCH_3$], compound No. 3 [formula (A): $R_1$ = CN, $R_2$ = $NH_2$, P = $COCH_3$] and compound No. 4 [formula (A): $R_1$ = CN, $R_2$ = $NH_2$, P = H]:

Scheme 2 shows the synthesis pathway. Namely, the above-mentioned compound No. 1 is converted into a compound represented by the formula (⑤), wherein $P_2$ and $P_3$ represent each a protective group, having protected hydroxyl groups (compound No. 2 when $P_2$ and $P_3$ are each a $COCH_3$ group). Next, the carbamoyl group of this compound is dehydrated to thereby give a compound of the formula (⑥) wherein $P_2$ and $P_3$ represent each a protective group (compound No. 3 when $P_2$ and $P_3$ are each a $COCH_3$ group). This compound is then deprived of the protective groups to thereby give the compound No. 4.

As the protective groups $P_2$ and $P_3$ of hydroxyl groups, those commonly used for protecting a hydroxyl group of a nucleic acid may be used. Examples thereof include acyl groups, for example, acetyl, chloroacetyl, dichloroacetyl and benzoyl groups, substituted and unsubstituted benzyl groups, and silyl groups, for example, trimethylsilyl, triethylsilyl and t-butyldimethyl silyl groups. It is preferable in the present invention to use an acyl group such as an acetyl group therefor.

The acylation may be performed under conventionally employed reaction conditions. In the case of acetylation, for example, the reaction may be effected in the presence of a basic solvent such as pyridine or triethylamine by using acetic anhydride, optionally further adding 4-dimethylaminopyridine used for acylating nucleic acids, at 0 to 50°C, preferably 10 to 40°C, for 4 to 48 hours, preferably 8 to 24 hours.

The carbamoyl group may be dehydrated with phosphorus oxychloride in the presence of triethylamine or with tosyl chloride in the presence of pyridine. It may be usually effected by treating the compound with phosphorus oxychloride in the presence of triethylamine under a nitrogen gas stream at -30 to 20°C, preferably -10 to 10°C, for 10 minutes to 3 hours, preferably 30 minutes to 2 hours.

The removal of the protective group may be conducted depending on the chemical properties of each protective group. For example, an acyl group, for example, acetyl group, employed in the present invention may be removed by treating with a concentrated aqueous ammonia or an aqueous solution of an alkali metal hydroxide such as NaOH or KOH, preferably concentrated aqueous ammonia, at 0 to 50°C, preferably at 10 to 40°C, for 10 minutes to 4 hours, preferably 30 minutes to 2 hours. Thus the compound No. 4 is obtained.

Each of these compounds is useful as an intermediate for synthesizing the compound No. 6. The hydroxyl groups of the compound No. 4 may be protected with appropriate protective groups, if required, to thereby give an intermediate for synthesizing the compound No. 6.

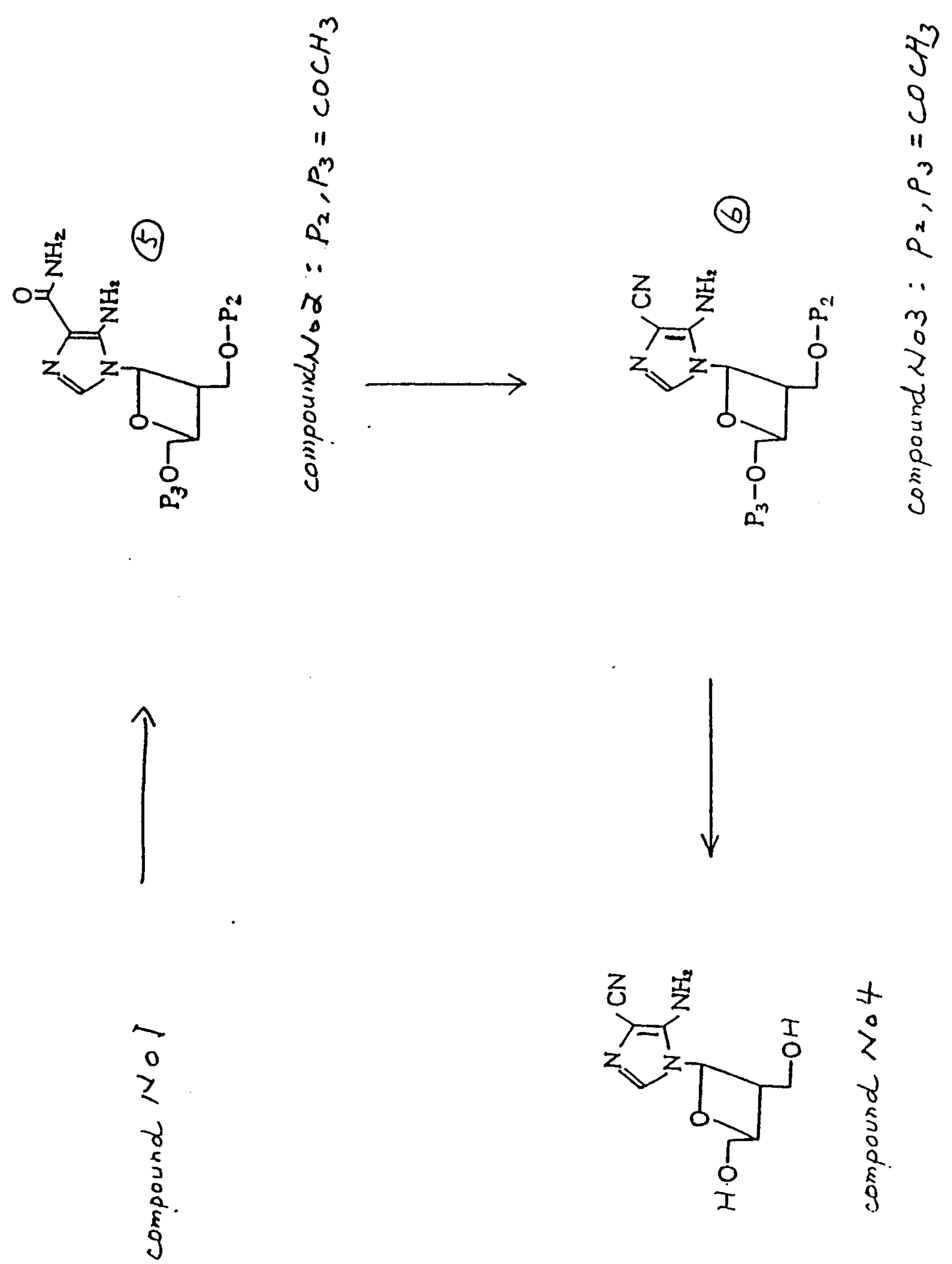

compound No 1

compound No 2 : $P_2$, $P_3$ = $COCH_3$

compound No 3 : $P_2$, $P_3$ = $COCH_3$

compound No 4

Scheme 2: Synthesis pathway of compound No. 4

3. Synthesis of compound No. 7 [formula (A): $R_1$ = CN, $R_2$ = I, P = $COCH_3$], compound No. 5 [formula (A): $R_1$ = CN, $R_2$ = -C=CH, p = $COCH_3$] and compound No. 6 [formula (A): $R_1$ = CN, $R_2$ = -C≡CH, P = H]:

The compound No. 6 may be synthesized from a compound of the above-mentioned formula (⑥) wherein $P_2$ and $P_3$ represent each a protective group via the pathway shown in Scheme 3. (Alternately, the protective groups for the hydroxyl groups of the isolated compound No. 4 may be changed.)

Namely, the amino group of the compound of the formula (⑥), wherein $P_2$ and $P_3$ represent each a protective group, is substituted with a halogen atom, preferably with an iodine atom, to thereby give a compound of the formula (⑦), wherein $P_2$ and $P_3$ represent each a protective group (compound No. 7 when $P_2$ and $P_3$ represent each a $COCH_3$ group). Next, an acetylene group substituted with a silyl group is introduced thereinto to thereby give a compound of the formula (⑧) wherein $P_2$ and $P_3$ represent each a protective group and Y is a silyl group. The silyl group of this compound is then removed to thereby give a

compound of the formula (⑨) wherein $P_2$ and $P_3$ represent each a protective group (compound No. 5 when $P_2$ and $P_3$ represent each a $COCH_3$ group). After removing the $P_2$ and $P_3$ groups, the compound No. 6 is obtained.

The above-mentioned iodination may be effected by reacting the compound of the formula (⑥) with an iodinating agent available as a solvent, for example, diiodomethane or methylene iodide, in the presence of an alkyl nitrite (for example, isoamyl nitrite or butyl nitrite) at 50 to 120° C, preferably 60 to 100° C, for 2 to 40 minutes, preferably 5 to 30 minutes.

Examples of the silyl group in the formula ( ⑧ ) include trimethylsilyl, t-butyldimethylsilyl and dimethylethylsilyl groups. In the present invention, trimethylsilyl group may be preferably used therefor.

The silyl-substituted acetylene group may be introduced by adding tributyltin trimethylsilylacetylene to the compound of the formula (⑦) by using a solvent, for example, acetonitrile, N,N-dimethylformamide, dimethyl sulfoxide or N,N-dimethylacetamide, in the presence of a palladium catalyst, for example, bis-(benzonitrile)palladium dichloride, bis(acetonitrile)palladium dichloride or bis(triphenylphosphine)palladium dichloride; preferably bis(benzonitrile)palladium dichloride. This reaction is effected in a hermetically sealed state at 50 to 150° C, preferably 80 to 120° C, for 4 to 24 hours, preferably for 6 to 12 hours.

The silyl group may be removed by the conventional method. For example, it may be removed by using tetrabutylammonium fluoride, ammonia fluoride or methanolic ammonia. On the other hand, the acyl group such as acetyl group may be removed with the use of methanolic ammonia or an aqueous solution of an alkali metal hydroxide, for example, NaOH or KOH. The silyl and acetyl groups of the compound of the formula ( ⑧ ) wherein $P_1$ and $P_2$ represent each a protective group and Y is a silyl group, may be simultaneously removed with the use of methanolic ammonia. In this method, the silyl group is removed by treating with tetrabutylammonium fluoride at 0 to 50° C, preferably 10 to 40° C, for 2 to 30 minutes, preferably 5 to 20 minutes to thereby give a compound of the formula (⑨) wherein $P_1$ and $P_2$ represent each a protective group. Next, acetyl groups of the obtained compound are removed by treating with methanolic ammonia at 0 to 50° C, preferably 10 to 40° C, for 10 minutes to 4 hours, preferably 30 minutes to 2 hours, thus synthesizing the compound No. 6.

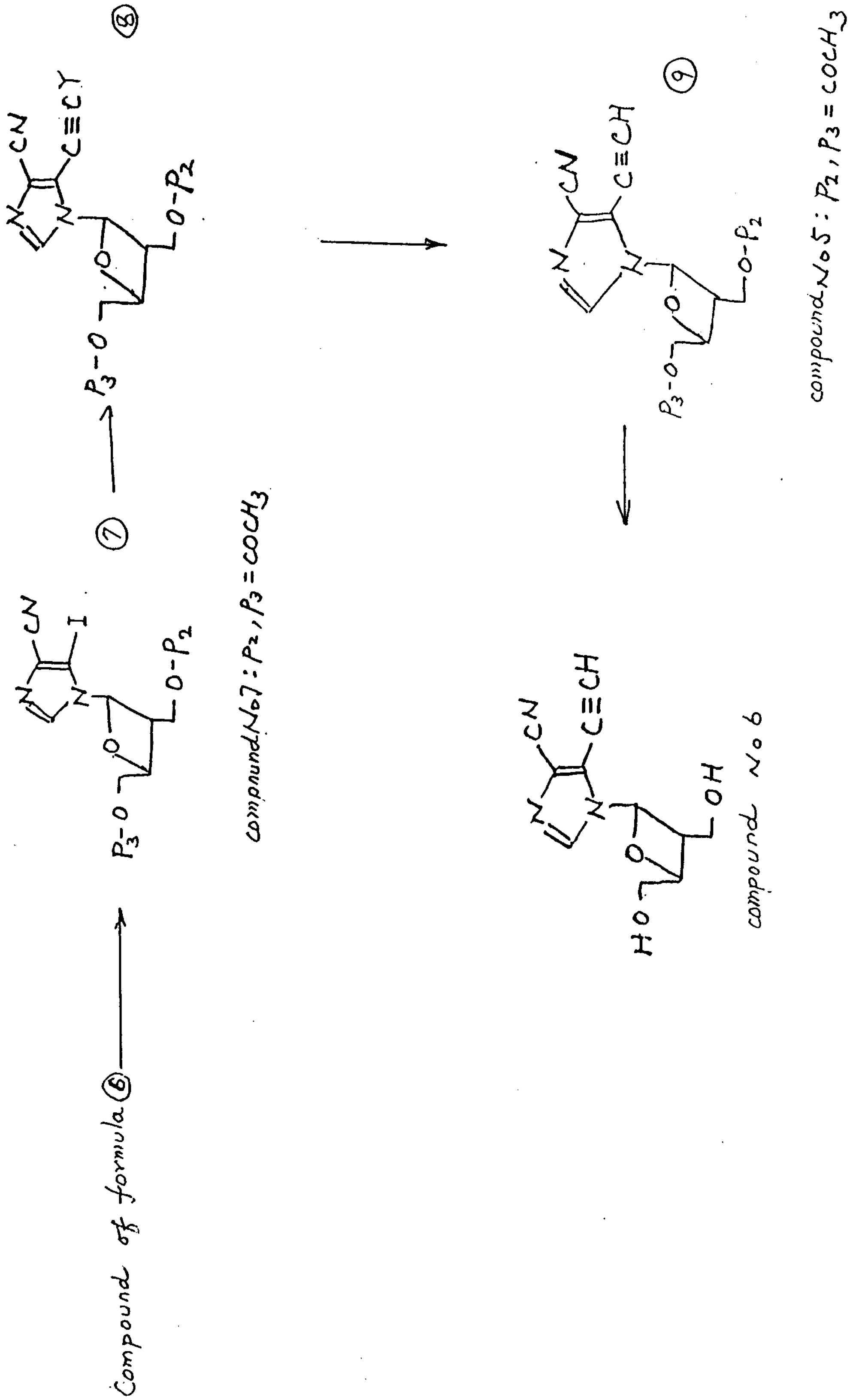

Compound of formula (6)

(7)

compound No 7 : $P_2$, $P_3$ = $COCH_3$

(8)

(9)

compound No 5 : $P_2$, $P_3$ = $COCH_3$

compound No 6

Scheme 3: Synthesis pathway of compound No. 6

The compound No. 6 [formula (A): $R_1$ = CN, $R_2$ = -C≡CH, P = H) may be used as a drug such as an immunosuppressant either alone or as a mixture thereof with adjuvants such as excipients or carriers in the form of, for example, an injection, an oral preparation or a suppository. The excipients and carriers are to be selected from among pharmaceutically acceptable ones. The type and composition thereof may be determined depending on the administration route and administration method. Examples of liquid carriers

include water, alcohols, animal and vegetable oils, e.g., soybean oil, peanut oil, rubber oil and mineral oils, and synthetic oils. Examples of solid carriers include sugars, e.g., maltose and sucrose, amino acids, cellulose derivatives, e.g., hydroxypropylcellulose, and organic acid salts, e.g., magnesium stearate. In the case of an injection, it is generally preferable to use physiological saline solution, various buffer solutions, solutions of sugars, e.g., glucose, inositol or mannitol, and glycols, e.g., ethylene glycol and polyethylene glycol. Alternately, the active compound may be lyophilized together with excipients such as sugars, e.g., inositol, mannitol, glucose, mannose, maltose or sucrose, or amino acids, e.g., phenylaianine. Then the lyophilized preparation thus obtained is dissolved in a solvent suitable for injection, for example, an intravenous administration solvent, e.g., sterilized water, physiological saline solution, a glucose solution, an electrolyte solution or an amino acid solution, prior to the administration.

The content of the compound of the present invention in a preparation may vary depending on the preparation. It may usually range from 0.1 to 100% by weight, preferably 1 to 90% by weight. For example, an injection may preferably contain 0.1 to 5% by weight of the compound of the present invention. In the case of oral administration, the active compound may be formulated into, for example, a tablet, a capsule, a dust, granules, a syrup or a dry syrup, together with the aforesaid solid or liquid carrier(s). The capsule, tablet or dust may usually contain approximately 3 to 100% by weight, preferably 5 to 90% by weight, of the compound of the present invention and the balance of carrier(s).

The administration dose may be determined depending on the age, body weight and conditions of the subject as well as the purpose of the treatment. In general, the dose may range from 1 to 300 mg/kg/day in the case of parenteral administration and from 5 to 500 mg/kg/day in the case of oral administration.

The compound No. 6 of the present invention may be formulated into a preparation by, for example, the following method. Purified water is added to 30 parts by weight of the compound in such a manner as to adjust the total amount to 2,000 parts by weight. After dissolving, the obtained solution is aseptically filtered through a Millipore filter of GS type. 2 g portions of the filtrate are introduced into 10 ml vials and lyophilized. Thus a lyophilized injection containing 30 mg of the compound per vial is obtained.

The compound of the present invention exerts an effect of suppressing the function of mouse lymphocytes which are immunocompetant cells. Namely, the effect of the compound on the blastogenesis of lymphocytes is examined in accordance with the method of Waithe et al. (cf. Waithe et al., Handbook of Experimental Immunology, page 26, Jan. 1978) As a result, this compound significantly suppresses the blastogenesis of T lymphocytes stimulated with Con A (concanavalin A) and the blastogenesis of B lymphocytes stimulated with LPS (lipopolysaccharide).

Now the pharmacological effects of the above-mentioned compound of the present invention will be described in greater detail.

Test Example 1

Suppression of blastogenesis of T lymphocytes with Con A:

BALB/ⓒ mouse spleen cells were pipetted into a microplate in such a manner as to give a concentration of 2 x $10^5$ cells/0.2 ml/well. Then the test compound at various concentrations was added to the wells except control ones. Further 5 $\mu$g/ml of Con A was added to all of the wells. Next, the cell suspensions were incubated in an incubator containing 5% carbon dioxide at 37°C for 72 hours. Eight hours prior to the completion of the incubation, 37 kBq/well of $^3$H-thymidine was added and the uptake thereof by the incubated cells was measured with a liquid scintillation counter. The $^3$H-thymidine uptake count of a lot to which Con A alone had been added was referred to as Adpm, while that of a lot to which Con A and the test compound had been added was referred to as Bdpm. Thus the value (1 - Bdpm/Adpm) x 100 was referred to as the suppression ratio of the test compound on blastogenesis. Table 1 summarizes the results.

Table 1

| Suppression of test compound on blastogenesis of T lymphocytes with Con A: | |
|---|---|
| Concentration of compound No. 6 ($\mu$g/ml) | Suppression ratio (%) |
| 0.006 | 33.1 |
| 0.024 | 36.4 |
| 0.10 | 34.0 |
| 0.39 | 50.0 |
| 1.56 | 81.8 |
| 6.25 | 90.3 |

As the above Table 1 clearly shows, the compound No. 6 intensely suppressed the blastogenesis of T lymphocytes.

Test Example 2

Suppression of blastogenesis of B lymphocytes with LPS:

Similar to the procedure of the Test Example 1 except that the Con A was replaced with 100 $\mu$g/ml of E. coli LPS, $^3$H-thymidine uptaken in blastogenic B cells was counted. The suppression ratio of the test compound was calculated by the same method as the one described above.

As Table 2 shows, the compound No. 6 intensely suppressed the blastogenesis of B lymphocytes with LPS.

Table 2

| Suppression of test compound on blastogenesis of B lymphocytes with LPS: | |
|---|---|
| Concentration of compound No. 6 ($\mu$g/ml) | Suppression ratio (%) |
| 0.10 | 0 |
| 0.39 | 1.5 |
| 1.56 | 22.8 |
| 6.25 | 91.4 |

These results indicate that the compound of the present invention suppresses the functions of B lymphocytes and T lymphocytes. These suppression effects respectively mean the suppression of humoral immunity and that of cellular immunity. Accordingly, it is strongly suggested that the compound of the present invention is applicable to the suppression of rejection in organ or skin transplantation, which might be mainly caused by the hyperstenia of humoral immunity and/or cellular immunity, as well as to the treatment of diseases mainly caused by autoimmunity, for example, multiple scieroma, hemolytic anemia, diabetes of type 1, serious myasthenia, Hashimoto's thyroiditis, Behcet's syndrome, rheumatism and allergic diseases.

Examples:

To further illustrate the present invention in greater detail, the following Examples will be given.

Example 1: Synthesis of compound No. 1 [formula (A):

$R_1$ = $CONH_2$, $R_2$ = $NH_2$, P = H]:

1-1. Synthesis of compound of formula (③) ($P_1$ = benzyl):

To a suspension of 56.3 g of oxetanocin A $N_1$-oxide (①) in 200 ml of N,N-dimethylacetamide was added 126.6 ml of benzyl bromide. Then the obtained solution was stirred at room temperature overnight. After adding 1.6 ℓ of water, the reaction mixture was extracted with 2 ℓ portions of chloroform twice and the aqueous phase was concentrated to dryness. To the syrup thus obtained [compound of formula (②): X = Br, $P_1$ = benzyl) was added 2.5 ℓ of a 0.67 N aqueous solution of sodium hydroxide, followed by stirring at room temperature overnight. Then the reaction mixture was washed with chloroform and neutralized with 1 N hydrochloric acid. Next, it was passed through a column packed with 800 ml of active carbon powder and washed with a 2% saline solution and water. After eluting with methanol, the eluate was concentrated to dryness to thereby give 58.6 g of the compound of the formula (③) ($P_1$ = benzyl).
MS m/z 348 $(M+H)^+$
Rf 0.53 ($CHCl_3$-MeOH=5:1)
$^1$H-NMR ($CD_3$ OD, ppm) 7.64 (1H, s) 、7.2~7. 5 (5H, m) 、6.01 (1H, d) 4.97 (2H, s) 、4.54 (1H, m) 、3.5~3.9 (5H, m) 。

1-2. Synthesis of compound No. 1:

To a mixture of 58.6 g of the compound of the formula (③) ($P_1$ = benzyl) dissolved in 800 ml of ethanol, 500 ml of water and 130 ml of acetic acid was added 5 g of 10% Pd-C. Then the mixture was subjected to hydrogenolysis at room temperature under 3 atm overnight. After filtering away the catalyst and concentrating the solvent, the residue [a compound of the formula (④)] was dissolved in 850 ml of concentrated aqueous ammonia and stirred at 100° C for 3 days in a sealed state. After concentrating to 600 ml, the reaction mixture was passed through a column packed with 500 ml of active carbon powder and washed with a 2% saline solution and water. After eluting with 30% aqueous methanol, the eluate was concentrated to dryness and crystallized from ethanol. Thus 7.2 g of the compound No. 1 (AICA-oxetanocin) was obtained.
MS m/a 243 $(M+H)^+$、265 $(M+Na)^+$
Rf 0.30 ($CHCl_3$-MeOH=3:1)
$^1$H-NMR ($D_2$ O, ppm) 7.60 (1H, s) 、6.02 (1H, d,) 、4.60 (1H, m) 、3.68~3.85 (4H, m) 、3.61 (1H, m)

Example 2: Synthesis of compound No. 2 [formula (A):

$R_1$ = $CONH_2$, $R_2$ = $NH_2$, P = $COCH_3$]:

To a suspension of 7.2 g of the compound No. 1 in 130 ml of N,N-dimethylformamide were added 180 mg of 4-dimethylaminopyridine, 17 ml of triethylamine and 6 ml of acetic anhydride. Then the obtained mixture was stirred at room temperature overnight. After distilling off the solvent under reduced pressure, the syrup thus obtained was separated by silica gel column chromatography (200 ml, chloroform : methanol = 20 : 1). Next, fractions around an Rf of 0.36 on silica gel TLC (developer : chloroform : methanol = 10 : 1) were collected and the solvent was distilled off under reduced pressure. Thus 9.6 g of the compound No. 2 was obtained.
MS m/z; 327 $(M+H)^+$
$^1$H-NMR ($CDCl_3$, ppm) 7.26 (1H, s) 、6.58 (1H, bs) 、5.94 (1H, d) 、5.43 (3H, bs) 、4.68 (1H, m): 4.22~4.45 (4H, m) 、3.69 (1H, m) 、2.11 、2.17 (6H, 2s)

Example 3: Synthesis of compound No. 3 [formula (A):

$R_1$ = CN, $R_2$ = $NH_2$, P = $COCH_3$]:

8 µl of triethylamine and 10.8 µl of phosphorus oxychloride were added to a solution of 38 mg of the compound No. 2 in 450 µl of chloroform under ice-cooling in a nitrogen gas stream. The obtained mixture was stirred at the same temperature for 50 minutes. After washing the reaction mixture with 500 µl of water, the solvent was concentrated under reduced pressure. The syrup thus obtained was then separated by silica gel column chromatography (20 ml, chloroform : methanol = 10 : 1). Then fractions around an Rf of

0.49 on silica gel TLC (developer : chloroform : methanol = 10 : 1) were collected and the solvent was distilled off under reduced pressure. Thus 25.1 mg of the compound No. 3 was obtained.
IR (film) $\tau$ = 3400 、2250 、1740 、1230cm$^{-1}$
$^1$H-NMR ($CDCl_3$ ppm) 7.38 (1H, s) 、5.95 (1H, d) 、4.73 (3H, m) 、4.34 (4H, m) 、3.61 (1H, m) 、2.15 、2.13 (6H, 2s)

Example 4: Synthesis of compound No. 4 [formula (A):

$R_1$ = CN, $R_2$ = $NH_2$, P = H]:

To a solution of 22 mg of the compound No. 3 in 0.5 ml of methanol was added 0.5 ml of concentrated aqueous ammonia and the obtained mixture was stirred at room temperature for 1 hour. Then the reaction mixture was concentrated to dryness under reduced pressure. The solid thus obtained was dissolved in 80% aqueous methanol and separated by column chromatography of Sephadex LH 20$^R$ (100 ml) equilibrated with the same solvent. Then fractions on silica gel TLC (developer : chloroform : methanol = 5 : 1) were collected. After distilling off the solvent under reduced pressure, 15.3 mg of the compound No. 4 was obtained.
Rf 0.3 ($CHCl_3$ -MeOH = 5:1)
MS m/z 225 $(M + H)^+$、247 $(M + Na)^+$
$^1$H-NMR ($D_2O$, ppm) 7.64 (1H, s) 、6.02 (1H, d) 、4.62 (1H, m) 、3.50~3.84 (5H, m)

Example 5: Synthesis of compound No. 6 [formula (A):

$R_1$ = CN, $R_2$ = -C≡CH, P = H]:

5-1. Synthesis of compound No. 7 [formula (⑦): $P_2$, $P_3$ = $COCH_3$]:

45 ml of diiodomethane was added to 1.1 g of the compound No. 3 and dissolved by stirring at 80 °C. Then 1.9 ml of isoamyl nitrite was added thereto and the mixture was stirred at the same temperature for 15 minutes. After cooling to room temperature, the reaction mixture was subjected to silica gel column chromatography (100 ml) and eluted with chloroform. Then fractions around an Rf of 0.70 on silica gel TLC (developer : chloroform : methanol = 20 : 1) were collected and the solvent was distilled off under reduced pressure. Thus 702 mg of the compound No. 7 [formula (⑦): $P_2$, $P_3$ = $COCH_3$] was obtained.
MS m/z 420 $(M + H)^+$
$^1$H-NMR ($CDCl_3$, ppm) 8.29 (1H,s), 6.08 (1H, d), 4.85 (1H, m) 4.24 ~ 4.45 (4H, m), 3.40 (1H, m), 2.16 (6H, 2s)

5-2. Synthesis of compound [formula (⑧): $P_2$, $P_3$ = $COCH_3$] Y = $Si(CH_3)_3$]:

86 mg of the compound [formula (⑦): $P_2$, $P_3$ = $COCH_3$] was dissolved in 3 ml of acetonitrile. Then 5.3 mg of bisbenzonitrilepalladium dichloride and 92 mg of tributyltin trimethylsilylacetylene were added thereto. The obtained mixture was stirred at 100 °C for 8 hours in a sealed tube. After distilling off the solvent, the obtained syrup was separated by silica gel column chromatography (50 ml, chloroform). Then fractions around an Rf of 0.79 on silica gel TLC (developer : chloroform : methanol = 15 : 1) were collected and the solvent was distilled off under reduced pressure. Thus 54.0 mg of the compound [formula (⑧): $P_2$, $P_3$ = $COCH_3$, Y = $Si(CH_3)_3$] was obtained.
M/S m/z 387 $(M + H)^+$
$^1$H-NMR ($CDCl_3$, ppm) 8.09 (1H, s), 6.19 (1H, d), 4.86 (1H, m), 4.35 (4H, m), 3.40 (1H, m), 2.13 (6H, s), 0.29 (9H, s)

5-3. Synthesis of compound No. 6:

67.7 mg of the compound [formula (⑧): $P_2$, $P_3$ = $COCH_3$, Y = $Si(CH_3)_3$] was dissolved in 5 ml of tetrahydrofuran. Then 170 µl of a 1 M solution of tetrabutylammonium fluoride in tetrahydrofuran was added thereto and the mixture was stirred at room temperature for 10 minutes. After distilling off the solvent, the

residue [compound No. 5 of the formula (⑨): $P_2$, $P_3$ = $COCH_3$] was dissolved in 6 ml of methanol. Then 2 ml of concentrated aqueous ammonia was added thereto, followed by stirring at room temperature for 1 hour. After distilling off the solvent, the obtained syrup was dissolved in 80% aqueous methanol and separated by silica gel column chromatography (Sephadex LH-20$^R$, 100 ml, equilibrated with the same solvent). Then fractions around an Rf of 0.40 on silica gel TLC (developer : chloroform : methanol = 5 : 1 ) were collected and the solvent was distilled off under reduced pressure. Thus 20.5 mg of the compound No. 6 was obtained.
MS m/z 233 ($M^+$)
$^1$H-NMR (DMSO-$d_6$, ppm) 8.65 (1H, s), 6.14 (1H, d), 5.41 (1H, s), 5.26 (1H, t), 5.07 (1H, t), 4.57 (1H, m), 3.40 ~ 3.74 (5H, m)

**Claims**

1. An oxetanocin derivative having an imidazole base group which is represented by the following general formula (A):

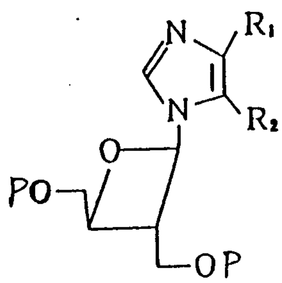

(A)

wherein $R_1$ represents a carbamoyl group or a cyano group;
$R_2$ represents an amino group, an acetylene group or a halogen atom; and
P represents a hydrogen atom or a protective group.

2. An oxetanocin derivative as claimed in Claim 1, wherein P is a hydrogen atom or an acyl group.

3. An oxetanocin derivative as claimed in Claim 1, wherein $R_1$ is a cyano group and $R_2$ is an acetylene group.

4. An oxetanocin derivative as claimed in Claim 1, wherein $R_1$ is a carbamoyl group and $R_2$ is an amino group.

5. An immunosuppressant consisting of a compound as claimed in any of Claims 1, 2 and 3 and pharmaceutical adjuvants.

European Patent Office

# EUROPEAN SEARCH REPORT

Application Number

EP 91 12 1765

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 291 917 (NIPPON KAYAKU K.K.)<br>* abstract *<br>--- | 1-5 | C07H19/02<br>A61K31/70 |
| Y | EP-A-0 155 164 (SUMITOMO PHARMACEUTICALS CO., LTD.)<br>* abstract *<br>--- | 1-5 | |
| P,A | EP-A-0 416 605 (NIPPON KAYAKU K.K.)<br>* abstract *<br>*page 4, compounds 3,4,5*<br>----- | 1 | |

TECHNICAL FIELDS SEARCHED (Int. Cl.5)

C07H
A61K

The present search report has been drawn up for all claims

| Place of search | Date of completion of the search | Examiner |
|---|---|---|
| THE HAGUE | 12 FEBRUARY 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document